# EUROPEAN PATENT SPECIFICATION

(11) **EP 1 799 146 B1**
(45) Date of publication and mention of the grant of the patent: **17.11.2010**
(21) Application number: 05797823.1
(22) Date of filing: 16.09.2005
(51) Int. Cl.: A61F 2/06, A61F 2/90, A61B 17/12

(54) **THIN FILM DEVICES FOR OCCLUSION OF A VESSEL**
DÜNNFILMVORRICHTUNGEN FÜR GEFÄSSVERSCHLUSS
DISPOSITIFS DE FILMS MINCES POUR UNE OCCLUSION DE VAISSEAU

(30) Priority: 17.09.2004 US 610781 P
(43) Date of publication of application: 27.06.2007
(73) Proprietor: Codman & Shurtleff, Inc., New Brunswick, NJ 08933 (US)
(72) Inventor: SHERMAN, Darren, R., Fort Lauderdale, FL 33301 (US); SLAZAS, Robert, R., Miami, FL 33176 (US)
(74) Representative: Gover, Richard Paul
(86) International application number: PCT/US2005/033327
(87) International publication number: WO 2006/034114

(56) References cited:
- WO-A-03/049600
- US-A- 6 096 175
- US-A1- 2001 039 449
- US-A1- 2005 197 687
- US-B1- 6 312 463
- US-B1- 6 312 463

## Description

This invention generally relates to medical devices that are implantable within a human subject and that have occlusion capabilities that are especially suitable for use as medical device plugs for defective or diseased body vessels. These types of devices have porosity characteristics, upon deployment, that are suitable for enhanced occlusion or other therapeutic capabilities at selected locations.

Medical devices that can benefit from the present invention include those that are characterized by hollow interiors and that are introduced endoluminally and expand when deployed so as to plug up a location of concern within the patient. These are devices that move between collapsed and expanded conditions or configurations for ease of deployment through catheters and introducers. The present disclosure focuses upon occlusion devices for diseased locations within vessels of the body, especially devices sized and configured for implantation within the vasculature, as well as devices for neurovascular use.

A number of technologies are known for fabricating implantable medical devices. Included among these technologies is the use of thin films. Current methods of fabricating thin films (on the order of several microns thick) employ material deposition techniques. These methods are known to make films into basic shapes, such as by depositing onto a mandrel or core so as to make thin films having the shape of the mandrel or core, such as geometric core shapes until the desired amount has built up. Traditionally, a thin film is generated in a simple (oftentimes cylindrical, conical, or hemispherical) form and heat-shaped to create the desired geometry. One example of a known thin film vapor deposition process can be found in Banas and Palmaz U.S. Patent Application Publication No. 2005/0033418.

Methods for manufacturing three-dimensional medical devices using planar films have been suggested, as in U.S. Patent No. 6,746,890 (Gupta et al.). The method described in Gupta et al. requires multiple layers of film material interspersed with sacrificial material. Accordingly, the methods described therein are time- consuming and complicated because of the need to alternate between film and sacrificial layers.

For some implantable medical devices, it is preferable to use a porous structure. Typically, the pores are added by masking or etching techniques or laser or water jet cutting. When occlusion devices are porous, especially for intercranial use, the pores are extremely small and these types of methods are not always satisfactory and can generate accuracy issues. Approaches such as those proposed by U.S. Patent Application publication No. 2003/0018381, include vacuum deposition of metals onto a deposition substrate which can include complex geometrical configurations. Microperforations are mentioned for providing geometric distendability and endothelialization. Such microperforations are said to be made by masking and etching or by laser-cutting.

An example of porosity in implantable grafts is disclosed in Boyle, Marton and Banas U.S. Patent Application Publication No. 2004/0098094. This publication proposes endoluminal grafts having a pattern of openings, and indicates that different orientations thereof could be practiced. Underlying stents support a microporous metallic thin film. Also, Schnepp-Pesch and Lindenberg U.S. Patent No. 5,540,713 describes an apparatus for widening a stenosis in a body cavity by using a stent-type of device having slots which open into diamonds when the device is radially expanded.

WO-03/049600 discloses a radially expandable device for use in the occlusion and repair of an undesired dilation in a vessel, such as aneurysm, while maintaining flow both through the vessel and through branches of the vessel that may be located in proximity to the aneurysm. This is achieved by having a device with a differential pore size wherein the portion of the device positioned in proximity to the aneurysm is of substantially smaller pore size than the portion of the device positioned on the side of the device away from the aneurysm.

US-6312463 discloses a prosthesis for treating a body passage including a microporous tubular element and support element. The microporous tubular element is formed from a thin walled sheet having a wall thickness of 25 micrometers or less. A mesh pattern within the tubular element includes a plurality of openings having a maximum dimension of not more than about 200 micrometers, thereby acting as a filter trapping embolic material while facilitating endothelial growth therethrough. The support element includes a plurality of struts.

A problem to be addressed is to provide an occlusion device with portions having reversible porosities that can be delivered endoluminally in surgical applications, and implanted and positioned at a desired location, wherein the porosities reverse from opened to closed or vice versa to provide an immediate occlusive function to "plug" the vessel defect and control or stop blood flow into the diseased site, and to provide a filtration function which allows adequate blood flow to reach adjacent perforator vessels.

Accordingly, an object of embodiments of the present invention is to provide occlusion devices having portions which perform a plugging function that substantially reduces or completely blocks blood flow to a diseased location of a blood vessel.

Another object of embodiments of this invention is to provide a method for plugging a vessel defect that can be performed in a single endoluminal procedure and that positions an occlusion device for effective blood flow control into and around the area of the diseased location.

Another object of embodiments of this invention is to provide an improved occlusion device that incorporates thin film metal deposition technology in preparing occlusion devices which have porosities which may include pore features that may move from opened to closed and vice versa.

Another object of embodiments of this invention is to provide an occlusion device which substantially reduces or blocks the flow of blood into or out of an aneurysm without completely preventing blood flow to other areas including adjacent perforator vessels or other features which can benefit from relatively low blood flow.

Other objects and advantages of the present invention, including the various features used in various combinations, will be understood from the following description according to preferred embodiments of the present invention, taken in conjunction with the drawings in which certain specific features are shown.

According to the present invention there is provided an expandable medical device having occlusion properties, comprising: an elongate carrying frame having a defined length, said frame being expandable from a collapsed condition to an expanded condition; a thin film mesh secured to said elongate carrying frame; and said thin film mesh has a plurality of openings therethrough that vary in degree of openness as said carrying frame moves between said collapsed condition and said expanded condition; **characterised in that** the thin film mesh is attached to the carrying frame by spring arms arranged to hold the thin film mesh taut and in place in the collapsed condition and in the expanded condition.

The carrying frame and the thin film mesh structure each have a contracted or collapsed pre-deployed configuration which facilitates endoluminal deployment as well as an expanded or deployed configuration within the body. When deployed within the body, the occlusion device is positioned so that the thin film mesh structure acts as a plug which substantially reduces or completely blocks blood flow to the diseased portion of the blood vessel. For example, the occlusion device is deployed so that the thin film mesh structure covers or plugs the neck of an aneurysm.

Porosity is provided in at least a portion of the thin film mesh structure in the radially contracted configuration in the form of pores or openings such as slots and/or slits that are either generally open or generally closed. In a preferred embodiment, at least some of the generally closed openings or pores open substantially, or at least some of them close substantially upon moving to the radially expanded or deployed configuration, typically resulting in longitudinal foreshortening of the thin film mesh structure.

In the embodiments where the openings or pores are open, or have opened, in the deployed configuration, the porosity is low enough to fully or partially occlude blood flow to a diseased portion of the vessel being treated, but large enough to allow passage of blood flow to adjacent perforator vessels. In the embodiments where the pores are substantially completely closed in the deployed configuration, the thin film mesh structure only extends over a portion of the deployed carrying frame, and the occlusion device is deployed so that the thin film mesh structure only covers as much tissue as necessary to plug the diseased portion of the blood vessel.

In making the thin film mesh, a core or mandrel is provided which is suited for creating a thin film by a physical vapor deposition technique, such as sputtering. A film material is deposited onto the core or mandrel to form a seemless or continuous three-dimensional layer. The thickness of the film will depend on the particular film material selected, conditions of deposition and so forth. Typically, the core then is removed by chemically dissolving the core, or by other known methods. Manufacturing variations allow the forming of multiple layers of thin film mesh material or a thicker layer of deposited material if desired. It is also contemplated that the thin film mesh structure could be made from a suitable plastically deformable material, such as stainless steel, platinum or other malleable metals, or a polymer.

Special application for the present invention has been found for creating porous occlusion devices which have a thin film mesh structure and selected porosity as deployed occlusion devices, and methods also are noted. However, it will be seen that the products and methods described herein are not limited to particular medical devices or methods of manufacture or particular surgical applications.
Fig. 1 is a front elevational view of an occlusion device in a collapsed configuration;
Fig. 2 is a front elevational view of the occlusion device of Fig. 1, in a deployed configuration within a blood vessel;
Fig. 3 is a front elevational view of an alternate occlusion device in a collapsed configuration;
Fig. 4 is a front elevational view of the occlusion device of Fig. 3, in a deployed configuration within a blood vessel;
Fig. 5 is a front elevational view of yet another alternate occlusion device in a collapsed configuration;
Fig. 6 is a front elevational view of the occlusion device of Fig. 5, in a deployed configuration within a blood vessel;
Fig. 7 is a front elevational view of yet another alternate occlusion device in a collapsed configuration;
Fig. 8 is a front elevational view of the occlusion device of Fig. 7, in the deployed configuration within a blood vessel;
Fig. 9 is a front elevational view of yet another alternate occlusion device;
Fig. 10 is a perspective view of yet another alternate occlusion device;
Fig. 11 is a perspective view of an occlusion device of an embodiment of the present invention, in a collapsed configuration; and
Fig. 12 is a perspective view of the occlusion device of Fig. 11, in a deployed configuration within a blood vessel.

### DESCRIPTION OF THE PREFERRED EMBODIMENTS

As required, detailed embodiments of the present invention are disclosed herein; however, it is to be understood that the disclosed embodiments are merely exemplary of the invention, which may be embodied in various forms. Therefore, specific details disclosed herein are not to be interpreted as limiting, but merely as a basis for the claims and as a representative basis for teaching one skilled in the art to variously employ the present invention in virtually any appropriate manner.

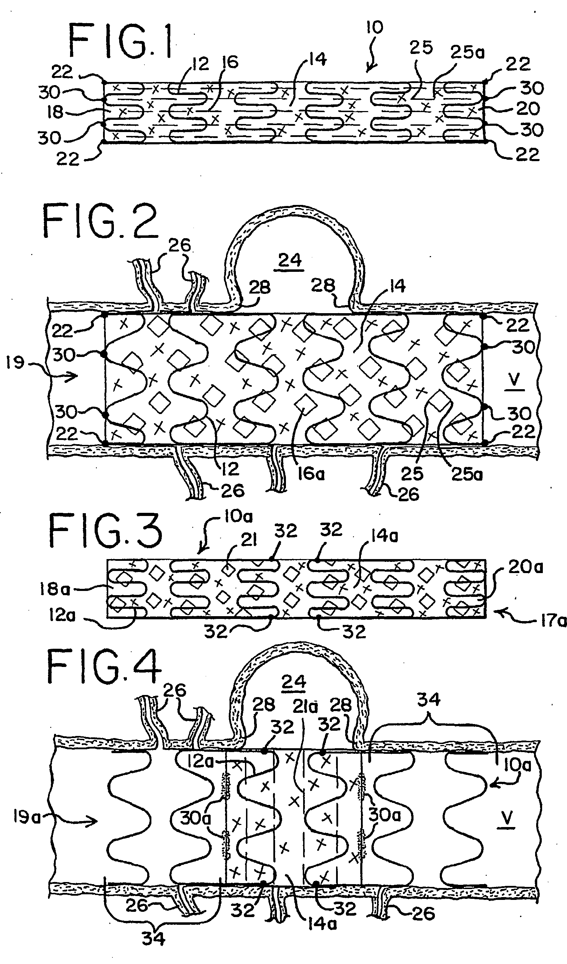

Fig. 1 illustrates an occlusion device 10 in a collapsed position. The occlusion device 10 comprises a carrying frame 12 and a thin film mesh structure 14 which extends over and attaches to the carrying frame 12. The thin film mesh structure 14 is preferably formed by physical vapor deposition onto a core or mandrel, as is generally known to those skilled in the art. Most preferably, a thin film mesh structure of nitinol, or other material which preferably has the ability to take on a shape that had been imparted to it during manufacture, is formed. When nitinol material is used in forming the thin film mesh structure 14, the thin film mesh structure can be at the martensite state. In addition, the mesh structure when made of nitinol or materials having similar shape memory properties may be austenite with a transition from martensite to austenite, typically when the device is raised to approximately human body temperature, or in the range of about 95 F. (35 C.) to 100 F (38 C.).

In making the thin film mesh structure 14, the selected material is sputter-deposited onto a core, which core is then removed by chemical etching or the like. Examples of this type of deposition are found in U.S. Published Patent Application No. 2003/0018381, No. 2004/0098094 and No. 2005/0033418. Nitinol, which encompasses alloys of nickel and titanium, is a preferred film material because of its superelastic and shape memory properties, but other known biocompatible compositions with similar characteristics may also be used. It is also contemplated that the thin film mesh structure can be made of a suitable plastically deformable material, such as stainless steel, platinum or other malleable metals, or a polymer.

The thickness of the thin film mesh structure, such as of structure 14, depends on the film material selected, the intended use of the device, the support structure, and other factors. For example, a thin film mesh structure of nitinol is preferably between about 0.1 and 250 microns thick and typically between about 1 and 30 microns thick. More preferably, the thickness of the thin film mesh structure is between about 1 to 10 microns or at least about 0.1 microns but less than about 5 microns.

The occlusion device 10 is shown in Fig. 1 in a collapsed configuration in which a plurality of pores or longitudinally extending slits 16 disposed at least along a portion of the thin film mesh structure 14 are substantially closed. The longitudinally extending slits 16 may be formed by any known means, but are preferably formed using laser-cutting. The slits 16 illustrated in Fig. 1 are shown in an identical patterned configuration, however the slits may assume differing profiles, e.g. curvilinear, and may be arranged randomly or in selected non-uniform patterns, according to the intended use.

The carrying frame 14 preferably comprises an expandable stent which may take on many different configurations and may be self-expandable or balloon expandable. Examples of such stents are disclosed in U.S. Patent Nos. 6,673,106 and 6,818,013, both to Mitelberg et al. Preferably the carry frame comprises an expandable stent which is laser cut from a tubular piece of nitinol. Alternatively, the carrying frame could also be a stent made from a suitable plastically deformable material, such as stainless steel, platinum or other malleable metals, or a polymer.

In the example illustrated in Figs. 1 and 2, the thin film mesh structure 14 covers the entire carry frame 12 in both the collapsed and expanded positions. In other words, the thin film mesh structure 14 substantially extends from one longitudinal end portion 18 of carry frame 12 to the other longitudinal end portion 20, and also extends 360 degrees around the carrying frame. To maintain full coverage of the carry frame 12, the thin film mesh structure 14 is tacked to the longitudinal end portions 18, 20 of the carry frame at locations generally designated 22. The thin film mesh structure 14 may be tacked to the carry frame 12 by weld, solder or adhesive. Although Fig. 1 illustrates tacking the thin film mesh structure 14 to the longitudinal end portions 18, 20 of the carrying frame 12, it will be understood that the thin film mesh structure 14 can be tacked at other locations along the carrying frame 12, depending on the desired use. Furthermore, it is contemplated that under certain situations it will be more advantageous for the thin film mesh structure 14 to line the interior of the carrying frame instead of covering the carrying frame.
As an alternative to tacking, the carrying frame 12 may be embedded or nested between separate layers of thin film mesh structure. This may be accomplished by sputtering a layer of thin film material onto a core. The carrying frame is then placed or formed over the core covered with thin film, and another layer of thin film can be sputtered over the thin film covered core carrying the carrying frame.

In use, the longitudinal slits 16 assist in allowing the occlusion device 10 to expand radially and foreshorten longitudinally. For example, Fig. 2 shows the occlusion device of Fig. 1 when same assumes a longitudinally foreshortened and radially expanded deployed configuration 19 within a body vessel V. When implanted in the body, the occlusion device 10, i.e. the carrying frame and the thin film mesh structure, moves from the elongated, collapsed configuration of Fig. 1 to the foreshortened, deployed configuration 19 of Fig. 2.

When the occlusion device has been deployed to the target area, the thin film mesh structure 14 expands radially, and the slits 16 of this embodiment move from the generally closed configuration slits 16 of Fig. 1 to the generally open configuration slots 16a of Fig. 2. The longitudinal ends 25, 25a of the slits 16 are compressed by the force of the occlusion device moving to its deployed configuration, causing the slits 16 to narrow and open, thereby contributing to having the thin film mesh structure 14 foreshorten and radially expand. In the open configuration, the slots 16a may assume a variety of open profiles, such as the illustrated diamond-shaped openings, depending on their initial closed profile. The open slots 16a are sized so that the thin film mesh structure 14 has a low porosity which substantially reduces or completely blocks the flow of blood into a diseased portion of a blood vessel, such as aneurysm 24. However, the open slots 16a are sized large enough to allow an adequate flow of blood to perforator vessels 26. Additionally, the open slots 16a can allow for tissue ingrowth and endothelialization for permanent fixation of the occlusion device.

The radially expanded configuration of the occlusion device as deployed in Fig. 2 is typically achieved by heating a carry frame made of a nitinol thin film mesh or other shape memory material when on a shaping core or mandrel until it reaches an austenite condition, whereby it is heat-set into the desired deployed shape and size. Furthermore, when the thin film mesh structure 14 is made from a nitinol or other shape memory material, it may be heat set in a similar fashion. The set shape of the carrying frame and the thin film mesh structure can be offset when cooled and removed from the mandrel and stretched down to a configuration such as shown in Fig. 1.

Typically, such memory "setting" is adequate to achieve the desired expanded or deployed shape of the device. However, the thin film mesh structure used in the occlusion device may be so thin as to provide very little expansion force or resistance to the expansive movement of the carrying frame 12. Thus, the outward expansive force of the carrying frame 12 may be the driver of the transition from the pre-deployed configuration to the deployed configuration of both the carrying frame 12 and the thin film mesh structure 14. It also can be possible to assist this expanded shaping by varying slot or slit size, shape, and location in both the carry frame and the thin film mesh structure.

For example, the elasticity of the thin film mesh structure can be supplemented in a desired area by overlapping portions of the thin film mesh structure with relatively large slits that telescope to allow for enhanced radial expansion when the occlusion device moves from a collapsed configuration to a deployed configuration.
Alternatively, if even less radial expansion is required, selected regions may be devoid of slits and slots, which means that the amount of expansion which results is due to the characteristics of the thin film material unaided by slots or slits in the material.
The occlusion device 10 is configured and sized for transport within a catheter or introducer of a delivery- system. A variety of delivery systems may be used to deploy the occlusion device within a vessel of a patient. The delivery system disclosed in U.S. Patent No. 6,833,003 to Jones et al. is particularly useful in delivering an occlusion device whose carry frame is a stent. In general, the occlusion device 10 is placed at a downstream end of a catheter, which catheter is introduced to the interior of a blood vessel V. The downstream end is positioned adjacent to a region of the blood vessel V which is to be occluded, and then a plunger or pusher member ejects the occlusion device into the target region. This may be achieved by moving the pusher member distally, moving the catheter in a retrograde direction, or a combination of both types of movement.
Preferably, the occlusion device 10 is comprised of a shape memory material, such as nitinol, which will move to a deployed configuration 19 upon exposure to living body temperatures, as shown in Fig. 2. Once the occlusion device 10 has been deployed, the catheter and plunger are thereafter removed from the vessel V, and the occlusion device is left at its deployed location.
The occlusion device 10 is deployed so that the thin film mesh structure 14 plugs or covers the neck 28 of the aneurysm 24. The open slots 16a are small enough to substantially reduce blood flow into or out of the aneurysm. This causes the blood within the aneurysm 24 to stagnate and form an occluding thrombus. Additionally, the open slots 16a are large enough to allow adequate blood flow to surrounding perforator vessels 26. It also should be noted that since the thin film mesh structure 14 covers the entire carrying structure 12, the deployment accuracy required may be less than with other prior art occlusion devices. However, the occlusion device 10 may also include radiopaque markers 30 to aid in proper deployment of the occlusion device.

According to an alternate example, referring to Figs. 3 and 4, the occlusion device 10a has a thin film mesh structure 14a which has a reversible porosity that is the opposite of the embodiment illustrated in Figs. 1 and 2. In other words, the thin film mesh structure 14a in the collapsed pre-deployed configuration has a plurality of open pores or slots 21 that close in the deployed configuration. These open slots 21 are preferably cut in an axial pattern along at least a portion of the thin film mesh structure 14a. Upon deployment, as illustrated in Fig. 4, the thin film mesh structure 14a expands radially and the slots 21 close into circumferentially oriented slits 21a as the thin film mesh structure 14a foreshortens. When the slots 21 are closed, the slits 21a are preferably at maximum density or fully closed to block the flow of blood from flowing into or out of a diseased portion of a blood vessel, such as aneurysm 24.

In the collapsed or pre-deployed configuration 17a, the thin film mesh structure 14a may cover the entire carrying frame 12a or a desired portion of the carrying frame 12a. Additionally, the thin film mesh structure 14a is tacked to the carrying frame at locations 32 which are substantially inward of the longitudinal end portions 18a and 20a of the carrying frame 12a. Tacking the thin film mesh structure 14a and the carrying frame 12a in this manner allows the thin film mesh structure to foreshorten more than the carrying frame when the occlusion device is in the deployed configuration 19a. This difference in foreshortening results in having portions 34 of the carrying frame 12a which are not covered by the thin film mesh structure 14a. Preferably, in the deployed configuration, the thin film mesh structure 14a covers between about 40% and about 60% of the carrying frame 12a. However, it is contemplated that the amount of coverage of the carry frame may greatly vary from this preferred amount depending on the intended use of the occlusion device.

In treating an aneurysm 24 within a blood vessel V of a patient, the occlusion device 10a may be delivered to the site of the aneurysm 24 using substantially the same deployment devices and deployment techniques as described above. In this example, the occlusion device 10a is deployed so that the expanded thin film mesh structure 14a having closed slots 18a covers only the neck 28 of the aneurysm 24 or an area slightly greater than the neck 28 of the aneurysm 24. The thin film mesh structure 14a may include radiopaque marks 30a to aid in deploying the occlusion device 10a to the desired location. The thin film mesh structure 14a plugs the aneurysm 24 and prevents blood from flowing into or out of the aneurysm, causing the creation of an occluding thrombus. Since the closed slotted thin film mesh structure 14a only covers the neck 28 of the aneurysm 24 or an area slightly larger than the neck 28 of the aneurysm 24, blood is allowed to flow through the uncovered portions 34 of the carrying frame 12a to provide an adequate blood supply to the perforator vessels 26.

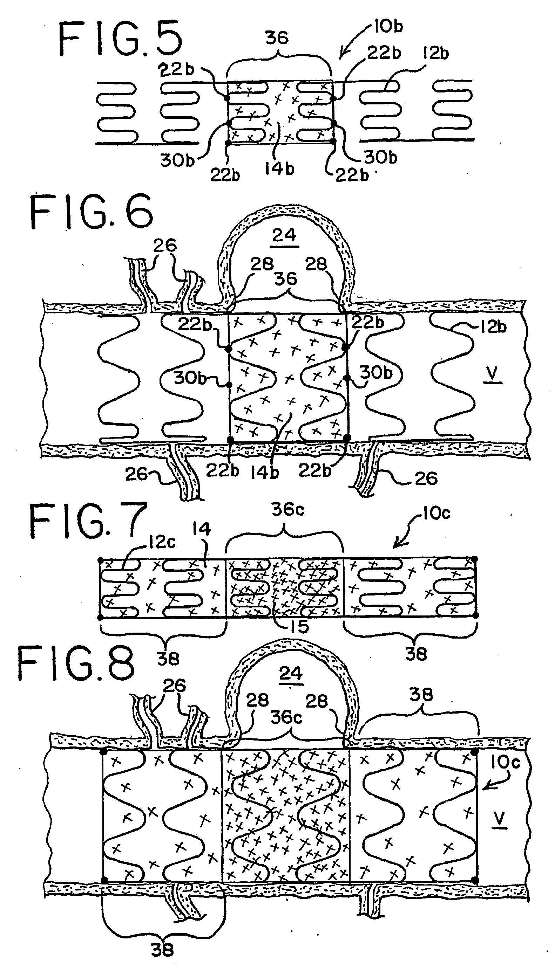

According to other alternative examples, referring to Figs. 5-10, the occlusion devices 10b, 10c, 10d and 10e include areas of high mesh density regions and areas of low mesh density regions. The term "mesh density" refers to the level of porosity or the ratio of metal to open area in a given portion of the device. A portion of the occlusion device which is considered a high mesh density region has approximately 40% or more metal area and about 60% or less open area. The mesh density, or ratio of metal area to open area, can be controlled by the number and size of the openings or pores and by the extent that the pores are open or closed in situations where opening or pore openness varies between delivery and deployment. It is preferred that the high mesh density area be generally longitudinally centered along the occlusion device, but it is also contemplated that the high mesh density area may be positioned anywhere along the occlusion device.

Referring specifically to Figs. 5 and 6, the high mesh density area 36 of the occlusion device 10b is created by centering a band of thin film mesh structure 14b on the carrying frame 12b so that the thin film mesh structure 14b extends 360 degrees around the carrying frame 12b but less than the full longitudinal extent of the device. The thin film mesh structure 14b is tacked to the carrying frame 12b at locations 22b. The thin illustrated film mesh structure 14b also includes radiopaque markers 30b to aid in aligning the high mesh density area in the desired location.

The occlusion device 10b is deployed to a blood vessel V of a patient so that the high mesh density area plugs a diseased portion of the blood vessel. For example, referring to Fig. 6, the occlusion device 10b is deployed so that the thin film mesh structure 14b providing a high mesh density area plugs the neck 28 of an aneurysm 24. The rest of the carry frame 12b is not covered by the thin film mesh structure 14b and thus allows blood to flow to the perforator vessels 26 or other areas thereat.

Referring to Figs. 7 and 8, the occlusion device 10c includes at least one portion of a high mesh density area 36c and at least one portion of a lower mesh density 38. There are a variety of different ways to construct the occlusion device 10c. For example, the occlusion device 10c may be constructed by covering the entire carrying frame 12c with a low density thin film mesh structure 14c and then adding an extra band of low density thin film mesh structure 15 around the center of the occlusion device to create an area 36c of high density thin film mesh structure. Another possible method would be to center a high density thin film mesh structure on the carrying frame, and then place low density bands of thin film mesh structure on the remaining uncovered portions of the carrying frame.

Referring to Fig. 8, the occlusion device 10c is deployed to a blood vessel V so that the high mesh density area 36c of the thin film mesh structure plugs the neck 28 of aneurysm 24. The lower mesh density area 38 of the thin film mesh structure preferably has a porosity that allows adequate blood flow to adjacent perforator blood vessels 26 or other areas adjacent this area 38.

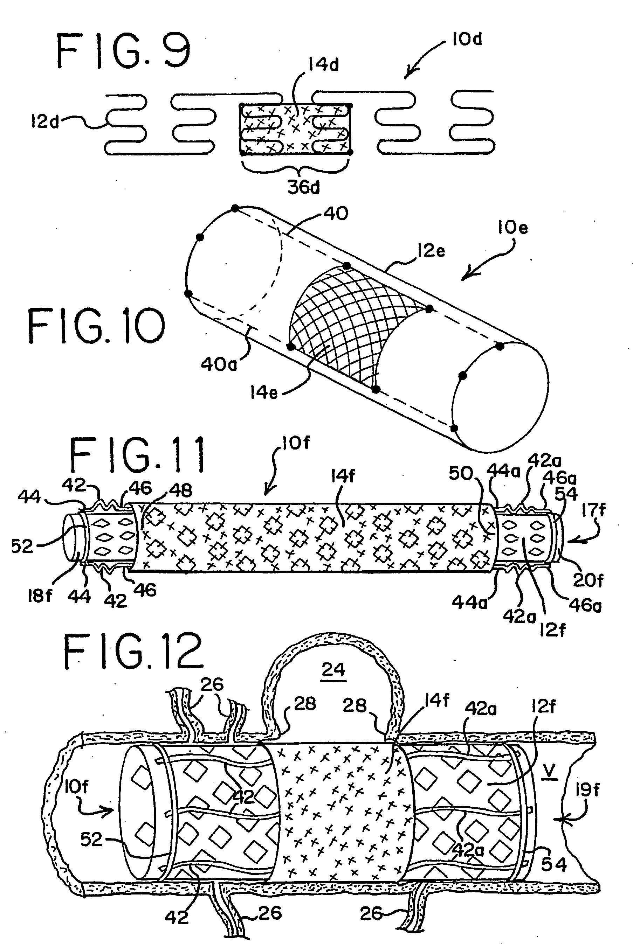

In yet another example of the occlusion device, referring to Fig. 9, a high mesh density area 36d is created by placing a patch 14d of thin film mesh structure on the carrying frame 12d. The longitudinal length of the patch 14d and the extent to which the patch 14d extends around the carrying frame 12d may vary greatly depending on the intended use of the occlusion device. It will be noted the patch extends for less than 360° of the circumference. In the illustrated example, this extends less than 180°, on the order of 120°.

Fig. 10 illustrates an alternate example of the carrying frame. In Fig. 10, the occlusion device 10e includes the carrying frame 12e which comprises a carrying frame which can be a stent formed from a wire frame. A patch 14e of thin film mesh structure is attached to wires portions 40 and 40a of the stent. As with the example of Fig. 9, the mesh structure is shown in Fig. 10 extending less than the full length and less than the full circumferential extent of the device.

An embodiment of the present invention is illustrated in Figs. 11 and 12. In this embodiment, the thin film mesh structure 14f is attached to the carrying frame 12f by spring arms 42 and 42a. The spring arms 42 and 42a are preferably strands of elastic material, such as nitinol or a polymer. Each spring arm 42 has a first longitudinal end 44 and a second longitudinal end 46. Each first longitudinal end 44 of spring arms 42 is attached to the first longitudinal end portion 18f of the carrying frame 12f, and each second longitudinal end 46 of the spring arms 42 is attached to the first longitudinal end portion 48 of the thin film mesh structure 14f. Similarly, the first longitudinal end 44a of each spring arm 42a is connected to the second longitudinal end portion 50 of the thin film mesh structure 14f, and the second longitudinal end 46a of each spring arm 42a is attached to the second longitudinal end portion 20f of the carrying frame 12f.

Preferably, each spring arm 42 and 42a is equally spaced apart from other adjacent spring arms around the occlusion device 10f. The spring arm, 42 and 42a may be attached to the carrying frame 12f and the thin film mesh structure 14f by weld, solder, biocompatible adhesive or other suitable biocompatible manner generally known in the art. In the illustrated embodiment, attachment includes using circumferential bands 52, 54, which may.take the form of shrink tubing or other type of banding, whether polymeric or metallic. Same can be radiopaque if desired.

As illustrated in Fig. 11, when the occlusion device 10f is in the collapsed or pre-deployed condition 17f, the spring arms 42 and 42a are in a collapsed position. In this collapsed position, the spring arms 42 and 42a are under tension to hold the thin film mesh structure 14f in place. Referring to Fig. 12, when the occlusion device 10f is in the deployed configuration 19f, the carrying frame 12f and the thin film mesh structure 14f expand radially, and the thin film mesh structure 14f foreshortens more than the carrying frame 12f. When the thin film mesh structure 14f is in the deployed configuration, the spring arms 42 and 42a are fully extended so as to hold the thin film mesh structure 14f taut and in-place.

When deployed in a blood vessel V of a patient to treat an aneurysm 24, the carrying frame 12f and the thin film mesh structure 14f expand radially, and the occlusion device 10f is positioned so that the thin film mesh structure 14f plugs or covers the neck 28 of the aneurysm 24, as illustrated in Fig. 12. As in the previous examples, the thin film mesh structure 14f may include radiopaque markers 30f to aid in deploying the occlusion device 10f into the desired position.

It will be understood that the embodiment of the present invention which has been described is illustrative of some of the applications of the principles of the present invention.

## Claims

1. An expandable medical device (10f) having occlusion properties, comprising:
an elongate carrying frame (12f) having a defined length, said frame (12f) being expandable from a collapsed condition to an expanded condition;
a thin film mesh (14f) secured to said elongate carrying frame (12f); and
said thin film mesh (14f) has a plurality of openings therethrough that vary in degree of openness as said carrying frame (12f) moves between said collapsed condition and said expanded condition; **characterised in that** the thin film mesh (14f) is attached to the carrying frame (12f) by spring arms (42, 42a) arranged to hold the thin film mesh (14f) taut and in place in the collapsed condition and in the expanded condition.

2. An expandable medical device (10f) according to claim 1, wherein the spring arms (42, 42a) comprise strands of elastic material.

3. An expandable medical device (10f) according to claim 1 or claim 2, wherein a first spring arm (42) extends between first ends of the carrying frame (12f) and the thin film mesh (14f) and a second spring arm (42a) extends between second ends of the carrying frame (12f) and the thin film mesh (14f).

4. An expandable medical device (10f) according to any one of the preceding claims, wherein each spring arm (42, 42a) is equally spaced apart from adjacent spring arms (42, 42a) around the medical device (10f).

5. The expandable medical device (10f) according to claim 1, wherein the thin film mesh (14f) is made of a shape memory material.

6. The expandable medical device (10f) according to claim 5, wherein the shape memory material comprises a nitinol.

7. The expandable medical device (10f) according to claim 1, wherein the carrying frame (12f) comprises a self-expanding carrying frame (12f).

8. The expandable medical device (10f) according to claim 1, wherein the carrying frame (12f) comprises a generally tubular stent having an inner and an outer surface.

9. The expandable medical device (10f) according to claim 1, wherein the plurality of openings include slits that open to slots as the carrying frame (12f) moves from said collapsed to said expanded condition.

10. The expandable medical device (10f) according to claim 1, wherein the plurality of openings include slots that close to slits as the carrying frame (12f) moves from said collapsed to said expanded condition.

11. The expandable medical device (10f) according to claim 1, wherein the thin film mesh (14f) has a thickness greater than about 0.1 microns but less than about 5 microns.

## Patentansprüche

1. Expandierbare medizinische Vorrichtung (10f) mit Verschlusseigenschaften, welche Folgendes umfasst:
einen länglichen Tragerahmen (12f) mit einer definierten Länge, wobei der Rahmen (12f) von einem zusammengelegten Zustand in einen expandierten Zustand expandierbar ist;
ein Dünnfilmgewebe (14f), welches an dem länglichen Tragerahmen (12f) befestigt ist; und
wobei das Dünnfilmgewebe (14f) mehrere Öffnungen dort hindurch aufweist, welche sich in einem Grad der Offenheit verändern, wenn sich der Tragerahmen (12f) zwischen dem zusammengelegten Zustand und dem expandierten Zustand bewegt;
**dadurch gekennzeichnet, dass** das Dünnfilmgewebe (14f) an dem Tragerahmen (12f) durch Federarme (42, 42a) angebracht ist, welche angeordnet sind, um das Dünnfilmgewebe (14f) in dem zusammengelegten Zustand und in dem expandierten Zustand straff und an der Stelle zu halten.

2. Expandierbare medizinische Vorrichtung (10f) gemäß Anspruch 1, wobei die Federarme (42, 42a) Stränge von elastischem Material umfassen.

3. Expandierbare medizinische Vorrichtung (10f) gemäß Anspruch 1 oder Anspruch 2, wobei sich ein erster Federarm (42) zwischen ersten Enden des Tragerahmens (12f) und dem Dünnfilmgewebe (14f) erstreckt und sich ein zweiter Federarm (42a) zwischen zweiten Enden des Tragerahmens (12f) und dem Dünnfilmgewebe (14f) erstreckt.

4. Expandierbare medizinische Vorrichtung (10f) gemäß einem der vorhergehenden Ansprüche, wobei jeder Federarm (42, 42a) von jedem benachbarten Federarm (42, 42a) um die medizinische Vorrichtung (10f) herum gleich beabstandet ist.

5. Expandierbare medizinische Vorrichtung (10f) gemäß Anspruch 1, wobei das Dünnfilmgewebe (14f) aus einem Material mit Formgedächtnis hergestellt ist.

6. Expandierbare medizinische Vorrichtung (10f) gemäß Anspruch 5, wobei das Material mit Formgedächtnis ein Nitinol umfasst.

7. Expandierbare medizinische Vorrichtung (10f) gemäß Anspruch 1, wobei der Tragerahmen (12f) einen selbstexpandierenden Tragerahmen (12f) umfasst.

8. Expandierbare medizinische Vorrichtung (10f) gemäß Anspruch 1, wobei der Tragerahmen (12f) einen im Allgemeinen röhrenförmigen Stent umfasst, welcher eine innere und eine äußere Oberfläche aufweist.

9. Expandierbare medizinische Vorrichtung (10f) gemäß Anspruch 1, wobei die mehreren Öffnungen Spalte aufweisen, welche sich in Schlitze öffnen, wenn sich der Tragerahmen (12f) von dem zusammengelegten in den expandierten Zustand bewegt.

10. Expandierbare medizinische Vorrichtung (10f) gemäß Anspruch 1, wobei die mehreren Öffnungen Schlitze aufweisen, welche sich zu Spalten schließen, wenn sich der Tragerahmen (12f) von dem zusammengelegten in den expandierten Zustand bewegt.

11. Expandierbare medizinische Vorrichtung (10f) gemäß Anspruch 1, wobei das Dünnfilmgewebe (14f) eine Dicke von mehr als etwa 0,1 µm aber weniger als etwa 5 µm aufweist.

## Revendications

1. Dispositif médical expansible (10f) ayant des propriétés occlusives, comprenant :
■ un châssis de support allongé (12f) ayant une longueur définie, ledit châssis (12f) étant expansible d'une condition repliée à une condition expansée ;
■ une maille de film fin (14f) fixée sur ledit châssis de support allongé (12f) ; et
■ ladite maille de film fin (14f) a une pluralité d'ouvertures à travers cette dernière qui varie en degrés d'ouverture au fur et à mesure que ledit châssis de support (12f) se déplace entre ladite condition repliée et ladite condition expansée ;
**caractérisé en ce que** la maille de film fin (14f) est fixée sur le châssis de support (12f) par des bras de ressort (42, 42a) agencés pour maintenir la maille de film fin (14f) tendue et en place dans la condition repliée et dans la condition expansée.

2. Dispositif médical expansible (10f) selon la revendication 1, dans lequel les bras de ressort (42, 42a) comprennent des brins de matériau élastique.

3. Dispositif médical expansible (10f) selon la revendication 1 ou la revendication 2, dans lequel un premier bras de ressort (42) s'étend entre les premières extrémités du châssis de support (12f) et la maille de film fin (14f) et un deuxième bras de ressort (42a) s'étend entre les deuxièmes extrémités du châssis de support (12f) et la maille de film fin (14f).

4. Dispositif médical expansible (10f) selon l'une quelconque des revendications précédentes, dans lequel chaque bras de ressort (42, 42a) est espacé à égale distance des bras de ressort (42, 42a) adjacents autour du dispositif médical (10f).

5. Dispositif médical expansible (10f) selon la revendication 1, dans lequel la maille de film fin (14f) est réalisée à partir d'un matériau à mémoire de forme.

6. Dispositif médical expansible (10f) selon la revendication 5, dans lequel le matériau à mémoire de forme comprend un Nitinol.

7. Dispositif médical expansible (10f) selon la revendication 1, dans lequel le châssis de support (12f) comprend un châssis de support auto-expansible (12f).

8. Dispositif médical expansible (10f) selon la revendication 1, dans lequel le châssis de support (12f) comprend un stent généralement tubulaire ayant une surface interne et une surface externe.

9. Dispositif médical expansible (10f) selon la revendication 1, dans lequel la pluralité d'oùvertures comprend des fentes qui s'ouvrent sur des rainures au fur et à mesure que le châssis de support (12f) passe de ladite condition repliée à ladite condition expansée.

10. Dispositif médical expansible (10f) selon la revendication 1, dans lequel la pluralité d'oùvertures comprend des rainures qui se ferment sur les fentes au fur et à mesure que le châssis de support (12f) passe de ladite condition repliée à ladite condition expansée.

11. Dispositif médical expansible (10f) selon la revendication 1, dans lequel la maille de film fin (14f) a une épaisseur supérieure à environ 0,1 micron, mais inférieure à environ 5 microns.
